# EUROPEAN PATENT APPLICATION

(11) **EP 4 383 187 A1**
(43) Date of publication of application: **12.06.2024**
(21) Application number: 22853483.0
(22) Date of filing: 04.08.2022
(51) Int. Cl.: G06T 5/00, G06T 7/73, G06T 17/20, G06T 15/08, G16H 30/40

(54) **METHOD AND DEVICE FOR NOISE FILTERING IN SCAN IMAGE PROCESSING OF THREE-DIMENSIONAL SCANNER**

(30) Priority: 04.08.2021 KR 20210102343
(71) Applicant: Medit Corp., Seoul 07207 (KR)
(72) Inventor: LEE, Dong Hoon, Seoul 07207 (KR); KANG, Dong Hwa, Seoul 07207 (KR)
(74) Representative: MFG Patentanwälte Meyer-Wildhagen Meggle-Freund Gerhard PartG mbB
(86) International application number: PCT/KR2022/011543
(87) International publication number: WO 2023/014107

(57) **Abstract**

Methods and devices for noise filtering of a three-dimensional scanner are disclosed. The scan image processing method according to one embodiment comprises: acquiring first scan data values regarding a surface of an object; acquiring second scan data values regarding the surface of the object; determining first vectors connecting the first scan data values from a virtual focus, or second vectors connecting the second scan data values from the virtual focus; determining whether first vectors intersect the second scan data values or second vectors intersect the first scan data values; and, if at least one of the first vectors intersects at least one of the second scan data values, deleting a data value associated with the intersected first vector, and, if at least one of the second vectors intersects at least one of the first scan data values, deleting the first scan data value that intersects with the second vector.

## Description

### TECHNICAL FIELD

The present disclosure relates to a method and a device for filtering noise in a scan image of a three-dimensional scanner. Specifically, the present disclosure relates to a method and a device for locally filtering noise present in a three-dimensional image model generated based on an image scanned by a scanner.

### BACKGROUND

A three-dimensional oral scanner is an optical device which is inserted into a patient's oral cavity to scan the teeth, enabling acquisition of a three-dimensional image of the oral cavity. Multiple two-dimensional images of the patient's mouth may be acquired by scanning the patient's oral cavity with the three-dimensional scanner, and the multiple two-dimensional images may be used to construct a three-dimensional image of the patient's oral cavity. For example, a dentist may insert a three-dimensional scanner into a patient's oral cavity to scan the patient's teeth, gums, and/or soft tissues, thereby acquiring multiple two-dimensional images of the patient's oral cavity. Then, by applying a three-dimensional modeling technique, the two-dimensional images of the patient's oral cavity may be used to construct a three-dimensional image of the patient's oral cavity.

However, when an object other than a subject to be scanned is interposed during such a scanning operation, for example, the dentist's finger or other treatment instrument is interposed between the scanner and a tooth while scanning the tooth, the portion of the tooth hidden by the interposed object is not scanned, and the interposed object is scanned instead.

Thus, the constructed three-dimensional image model will have noise images caused by the interposed object. Since the presence of noise makes it impossible to acquire an accurate model of a desired subject, it is necessary to effectively remove such noise when constructing a three-dimensional image model.

### SUMMARY

The present disclosure is intended to solve the above problems in the conventional art, and enables effective removal of noise that may be present in a three-dimensional image model generated using a three-dimensional scanner.

As one aspect of the present disclosure, a method for filtering noise in a scan image of a three-dimensional scanner may be suggested. A method according to one aspect of the present disclosure is a method for processing a scan image of a three-dimensional scanner, which is performed by at least one processor of an electronic device comprising the at least one processor and at least one memory configured to store instructions to be executed by the at least one processor, wherein the method may comprises: acquiring first scan data values regarding a surface of a subject by a first scan of the three-dimensional scanner, the first scan data values comprising a three-dimensional coordinate values; acquiring second scan data values regarding the surface of the subject by a second scan of the three-dimensional scanner, the second scan data values comprising a three-dimensional coordinate values; determining first vectors connecting a virtual focal point of the three-dimensional scanner to the first scan data values or second vectors connecting the virtual focal point to the second scan data values; determining whether the first vectors intersect the second scan data values or whether the second vectors intersect the first scan data values; and in case that at least one of the first vectors intersects at least one of the second scan data values, deleting a data value, among the first scan data values, which is associated with the at least one first vector intersecting the at least one second scan data value, and in case that at least one of the second vectors intersects at least one of the first scan data values, deleting a data value, among the first scan data values, which intersects the at least one second vector.

As one aspect of the present disclosure, an electronic device for filtering noise in a scan image of a three-dimensional scanner may be suggested. An electronic device according to one aspect of the present disclosure may comprise: a communication circuit communicatively connected to a three-dimensional scanner; a display; and at least one processor, wherein the at least one processor is configured to: acquire first scan data values regarding a surface of a subject by a first scan of the three-dimensional scanner, the first scan data values comprising three-dimensional coordinate values; acquire second scan data values regarding the surface of the subject by a second scan of the three-dimensional scanner, the second scan data values comprising three-dimensional coordinate values; determine first vectors connecting a virtual focal point of the three-dimensional scanner to the first scan data values or second vectors connecting the virtual focal point to the second scan data values; determine whether the first vectors intersect the second scan data values or whether the second vectors intersect the first scan data values; and in case that at least one of the first vectors intersects at least one of the second scan data values, delete a data value, among the first scan data values, which is associated with the at least one first vector intersecting the at least one second scan data value, and in case that at least one of the second vectors intersects at least one of the first scan data values, delete a data value, among the first scan data values, which intersects the at least one second vector.

As one aspect of the present disclosure, a non-transitory computer-readable recording medium storing instructions for filtering noise in a scan image of a three-dimensional scanner may be suggested. A non-transitory computer-readable recording medium, according to one aspect of the present disclosure, is a non-transitory computer-readable recording medium storing instructions which, when executed by at least one processor, cause the at least one processor to perform operations, wherein the instructions may cause the at least one processor to: acquire first scan data values regarding a surface of a subject by a first scan of a three-dimensional scanner, the first scan data values comprising three-dimensional coordinate values; acquire second scan data values regarding the surface of the subject by a second scan of the three-dimensional scanner, the second scan data values comprising three-dimensional coordinate values; determine first vectors connecting a virtual focal point of the three-dimensional scanner to the first scan data values or second vectors connecting the virtual focal point to the second scan data values; determine whether the first vectors intersect the second scan data values or whether the second vectors intersect the first scan data values; and in case that at least one of the first vectors intersects at least one of the second scan data values, delete a data value, among the first scan data values, which is associated with the at least one first vector intersecting the at least one second scan data value, and in case that at least one of the second vectors intersects at least one of the first scan data values, delete a data value, among the first scan data values, which intersects the at least one second vector.

As one aspect of the present disclosure, a system for three-dimensional scanning may be suggested. A system for three-dimensional scanning according to one aspect of the present disclosure may comprise: a three-dimensional scanner configured to scan a shape of an oral cavity; and an electronic device communicably coupled to the three-dimensional scanner, wherein the electronic device comprises: a communication circuit communicatively connected to the three-dimensional scanner; and at least one processor, wherein the at least one processor is configured to: acquire first scan data values regarding a surface of a subject by a first scan of the three-dimensional scanner, the first scan data values comprising three-dimensional coordinate values; acquire second scan data values regarding the surface of the subject by a second scan of the three-dimensional scanner, the second scan data values comprising three-dimensional coordinate values; determine first vectors connecting a virtual focal point of the three-dimensional scanner to the first scan data values or second vectors connecting the virtual focal point to the second scan data values; determine whether the first vectors intersect the second scan data values or whether the second vectors intersect the first scan data values; and in case that at least one of the first vectors intersects at least one of the second scan data values, delete a data value, among the first scan data values, which is associated with the at least one first vector intersecting the at least one second scan data value, and in case that at least one of the second vectors intersects at least one of the first scan data values, delete a data value, among the first scan data values, which intersects the at least one second vector.

According to at least an embodiment of the method and the device for processing a scan image in the present disclosure, scan data values acquired by a first scan and scan data values acquired by a second scan may be used to effectively remove noise present in the scan data values acquired by the first scan.

According to at least an embodiment of the method and the device for processing a scan image in the present disclosure, the accuracy of a three-dimensional image model of a desired subject may be improved by removing noise present in the scan data values acquired by the first scan.

### BRIEF DESCRIPTION OF DRAWINGS

FIG. 1 illustrates a view showing acquisition of an image of a patient's oral cavity using a three-dimensional scanner according to various embodiments of the present disclosure.
FIG. 2A is a block diagram of a three-dimensional scanner and an electronic device according to various embodiments of the present disclosure. FIG. 2B is a perspective view of the three-dimensional scanner according to various embodiments of the present disclosure.
FIG. 3 illustrates a method for generating a three-dimensional image of an oral cavity according to various embodiments of the present disclosure.
FIGS. 4A to 4E are schematic views illustrating situations in which noise is generated during scanning using a three-dimensional scanner.
FIG. 5 is a flowchart of a noise filtering method according to various embodiments of the present disclosure.
FIGS. 6A to 6D are schematic views illustrating a configuration for filtering noise based on the noise filtering method in FIG. 5.
FIG. 7 is a flowchart of a noise filtering method according to various embodiments of the present disclosure.
FIGS. 8A to 8D illustrate a noise filtering method according to various embodiments of the present disclosure.
FIGS. 9A to 9D illustrate a noise filtering method according to various embodiments of the present disclosure.
FIGS. 10A and 10B illustrate results of noise filtering performed according to various embodiments of the present disclosure.

### DETAILED DESCRIPTION

Embodiments of the present disclosure are illustrated for describing the technical idea of the present disclosure. The scope of the claims according to the present disclosure is not limited to the embodiments described below or to the detailed descriptions of these embodiments.

All technical or scientific terms used in the present disclosure have meanings that are generally understood by a person having ordinary knowledge in the art to which the present disclosure pertains, unless otherwise specified. The terms used in the present disclosure are selected for the purpose of clearer explanation of the present disclosure, and are not intended to limit the scope of claims according to the present disclosure.

The expressions "include," "provided with," "have" and the like used in the present disclosure should be understood as open-ended terms connoting the possibility of inclusion of other embodiments, unless otherwise mentioned in a phrase or sentence including the expressions.

A singular expression used in the present disclosure can include meanings of plurality, unless otherwise mentioned, and the same is applied to a singular expression stated in the claims. The terms "first," "second," etc. used the present disclosure are used to distinguish a plurality of elements from one another, and are not intended to limit the order or importance of the relevant elements.

The term "unit" used in the present disclosure means a software element or hardware element, such as a field-programmable gate array (FPGA) or an application specific integrated circuit (ASIC). However, a "unit" is not limited to software and hardware. A "unit" may be configured to be stored in an addressable storage medium or may be configured to run on one or more processors. Therefore, for example, a "unit" may include elements, such as software elements, object-oriented software elements, class elements, and task elements, as well as processors, functions, attributes, procedures, subroutines, segments of program codes, drivers, firmware, micro-codes, circuits, data, databases, data structures, tables, arrays, and variables. Functions provided in elements and "unit" may be combined into a smaller number of elements and "units" or further subdivided into additional elements and "units."

The expression "based on" used in the present disclosure is used to describe one or more factors that influences a decision, an action of determination, or an operation described in a phrase or sentence including the relevant expression, and this expression does not exclude an additional factor influencing the decision, the action of determination, or the operation.

In the present disclosure, when a certain element is described as being "coupled to" or "connected to" another element, it should be understood that the certain element may be connected or coupled directly to the other element or that the certain element may be connected or coupled to the other element via a new intervening element.

In the present disclosure, artificial intelligence (AI) may refer to a technology that imitates human learning ability, reasoning ability, and perception ability and implements the same with a computer, and may include concepts of machine learning and symbolic logic. Machine learning (ML) may be an algorithmic technique that automatically classifies or learns the characteristics of input data. Artificial intelligence technology may use machine learning algorithms to analyze input data, learn the results of the analysis, and make determinations or predictions based on the results of the learning. Further, technologies that mimic the cognitive and decision-making functions of the human brain by using machine learning algorithms may also be considered to fall within the realm of artificial intelligence. For example, technical fields of language understanding, visual comprehension, reasoning and prediction, knowledge representation, and action control may be included.

In the present disclosure, machine learning may refer to a process of training a neural network model by using experience in processing data. Machine learning may imply that computer software improves its ability to process data on its own. A neural network model is constructed by modeling correlations between data, wherein the correlations may be represented by multiple parameters. The neural network model extracts features from given data and analyzes the features to derive correlations between data, and repeating this process to optimize the parameters of the neural network model may be referred to as machine learning. For example, a neural network model can learn the mapping (correlation) between inputs and outputs with respect to data provided as input-output pairs. Alternatively, even when only input data is provided, a neural network model can learn the relationships between the provided data by deriving regularities in the data.

In the present disclosure, an artificial intelligence learning model, a machine learning model, or a neural network model may be designed to implement a human brain structure on a computer and may include multiple network nodes that mimic neurons in a human neural network and have weights. Multiple network nodes may have connections to one another by mimicking the synaptic activity of neurons sending and receiving signals through synapses. In an artificial intelligence learning model, multiple network nodes can be located on layers having different depths to send and receive data based on convolutional connections. The artificial intelligence learning model may be, for example, an artificial neural network, a convolutional neural network, etc.

Hereinafter, embodiments of the present disclosure will be described with reference to the accompanying drawings. In the accompanying drawings, identical or corresponding elements are indicated by identical reference numerals. In the following description of embodiments, repeated descriptions of the identical or corresponding elements will be omitted. However, even when a description of an element is omitted, such an element is not intended to be excluded in an embodiment.

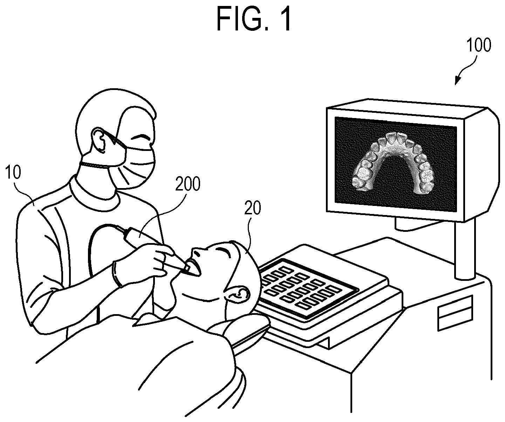

FIG. 1 illustrates acquiring an image of a patient's oral cavity by using a three-dimensional scanner 200 according to various embodiments of the present disclosure. According to various embodiments, the three-dimensional scanner 200 may be a dental medical device for acquiring an image of an oral cavity of a subject 20. For example, the three-dimensional scanner 200 may include an intraoral scanner. As illustrated in FIG. 1, a user 10 (e.g., a dentist or a dental hygienist) may use the three-dimensional scanner 200 to acquire an image of the oral cavity of a subject 20 (e.g., a patient) from the subject 20. In another example, the user 10 may acquire an image of the oral cavity of the subject 20 from a diagnostic model (e.g., a plaster model or an impression model) that is made in the shape of the oral cavity of the subject 20. Hereinafter, for ease of explanation, it will be described that an image of the oral cavity of the subject 20 is acquired by scanning the oral cavity of the subject 20. However, the present disclosure is not limited thereto, and it is also possible to acquire an image of another part of the subject 20 (e.g., an ear of the subject 20). The three-dimensional scanner 200 may be shaped to be inserted into or removed from the oral cavity, and may be a handheld scanner for which a scanning distance and a scanning angle can be freely adjusted by the user 10.

The three-dimensional scanner 200 according to various embodiments may be inserted into the oral cavity of the subject 20 and scan the interior of the oral cavity in a non-contact manner, thereby acquiring an image of the oral cavity. The image of the oral cavity may include at least one tooth, gingiva, and artificial structures insertable into the oral cavity (e.g., orthodontic devices including brackets and wires, implants, dentures, and orthodontic aids inserted into the oral cavity). The three-dimensional scanner 200 may use a light source (or a projector) to emit light to the oral cavity of the subject 20 (e.g., at least one tooth or gingiva of the subject 20), and may receive light reflected from the oral cavity of the subject 20 through a camera (or at least one image sensor). According to another embodiment, the three-dimensional scanner 200 may scan a diagnostic model of the oral cavity to acquire an image of the diagnostic model of the oral cavity. When the diagnostic model of the oral cavity is a diagnostic model that mimics the shape of the oral cavity of subject 20, an image of the diagnostic model of the oral cavity may be an image of the oral cavity of the subject. For ease of explanation, the following description assumes, but is not limited to, acquiring an image of the oral cavity by scanning the inside of the oral cavity of the subject 20.

The three-dimensional scanner 200 according to various embodiments may acquire a surface image of the oral cavity of the subject 20 as a two-dimensional image based on information received through a camera. The surface image of the oral cavity of the subject 20 may include at least one among at least one tooth, gingiva, artificial structure, cheek, tongue, or lip of the subject 20. The surface image of the oral cavity of subject 20 may be a two-dimensional image.

The two-dimensional image of the oral cavity acquired by the three-dimensional scanner 200 according to various embodiments may be transmitted to an electronic device 100 which is connected via a wired or wireless communication network. The electronic device 100 may be a computer device or a portable communication device. The electronic device 100 may generate a three-dimensional image (or, three-dimensional oral cavity image or a three-dimensional oral model) of the oral cavity, which is a three-dimensional representation of the oral cavity, based on the two-dimensional images of the oral cavity received from the three-dimensional scanner 200. The electronic device 100 may generate the three-dimensional image of the oral cavity by modeling the inner structure of the oral cavity in three dimensions, based on the received two-dimensional images of the oral cavity.

The three-dimensional scanner 200 according to another embodiment may scan the oral cavity of the subject 20 to acquire two-dimensional images of the oral cavity, generate a three-dimensional image of the oral cavity based on the acquired two-dimensional images of the oral cavity, and transmit the generated three-dimensional image of the oral cavity to the electronic device 100.

The electronic device 100 according to various embodiments may be communicatively connected to a cloud server (not shown). In this case, the electronic device 100 may transmit two-dimensional images of the oral cavity of the subject 20 or a three-dimensional image of the oral cavity to the cloud server, and the cloud server may store the two-dimensional images of the oral cavity of the subject 20 or the three-dimensional image of the oral cavity, which has been received from the electronic device 100.

According to another embodiment, in addition to a handheld scanner that is inserted into the oral cavity of subject 20, a table scanner (not shown) which is fixed and used in a specific location may be used as the three-dimensional scanner. The table scanner can generate a three-dimensional image of a diagnostic model of the oral cavity by scanning the diagnostic model of the oral cavity. In the above case, since a light source (or projector) and a camera of the table scanner are fixed, the user may scan the oral diagnosis model while moving an arm for fixing the oral diagnosis model. In comparison to a handheld scanner, although the table scanner may have a relatively lower possibility of causing noise due to intervention by other objects between the camera and the diagnosis model during the scanning operation, the embodiments of the present disclosure are applicable not only to handheld scanners, but also to table scanners and other three-dimensional scanners.

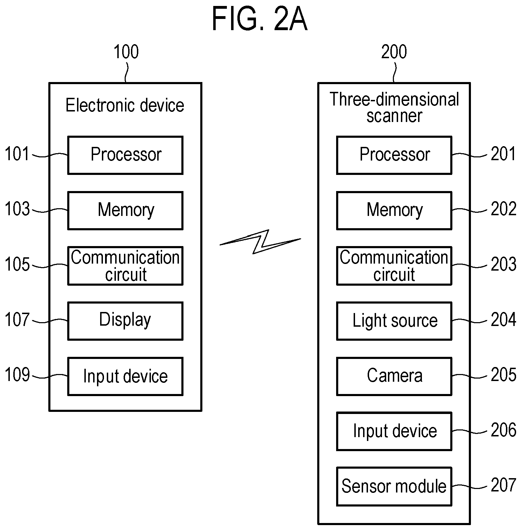

FIG. 2A is a block diagram of an electronic device 100 and a three-dimensional scanner 200 according to various embodiments of the present disclosure. The electronic device 100 and the three-dimensional scanner 200 may be communicatively connected to each other via a wired or wireless communication network, and may transmit and receive various types of data to and from each other.

The three-dimensional scanner 200 according to various embodiments may include a processor 201, a memory 202, a communication circuit 203, a light source 204, a camera 205, an input device 206, and/or a sensor module 207. At least one of the elements included in the three-dimensional scanner 200 may be omitted, or other elements may be added to the three-dimensional scanner 200. Additionally or alternatively, some elements may be integrated, or implemented as a single or multiple entities. At least some elements in the three-dimensional scanner 200 may be connected to each other via a bus, a general purpose input/output (GPIO), a serial peripheral interface (SPI), or a mobile industry processor interface (MIPI) so as to transmit and/or receive data and/or signals.

The processor 201 of the three-dimensional scanner 200 according to various embodiments is an element capable of performing operations or data processing related to control and/or communication of the elements of the three-dimensional scanner 200, and may be operatively coupled to the elements of the three-dimensional scanner 200. The processor 201 may load commands or data received from other elements of the three-dimensional scanner 200 into the memory 202, may process the commands or data stored in the memory 202, and may store resulting data. According to various embodiments, the memory 202 of the three-dimensional scanner 200 may store instructions for the above-described operations of the processor 201.

According to various embodiments, the communication circuit 203 of the three-dimensional scanner 200 may establish a wired or wireless communication channel with an external device (e.g., the electronic device 100), and may transmit and receive various types of data to and from the external device. According to an embodiment, for wired communication with the external device, the communication circuit 203 may include at least one port to be connected to the external device with a wired cable. In the above case, the communication circuit 203 may communicate with the external device that is connected to a wire via the at least one port. According to an embodiment, the communication circuit 203 may be configured to include a cellular communication module so as to be connected to a cellular network (e.g., 3G, LTE, 5G, Wibro, or Wimax). According to various embodiments, the communication circuit 203 may include a short-range communication module to transmit and receive data to and from external devices by using short-range communication (e.g., Wi-Fi, Bluetooth, Bluetooth Low Energy (BLE), UWB), but the present disclosure is not limited thereto. According to an embodiment, the communication circuit 203 may include a contactless communication module for contactless communication. The contactless communication may include at least one contactless proximity communication technology, for example, near-field communication (NFC) communication, radio frequency identification (RFID) communication, or magnetic secure transmission (MST) communication.

According to various embodiments, the light source 204 of the three-dimensional scanner 200 may emit light toward the oral cavity of the subject 20. For example, the light emitted from the light source 204 may be structured light having a predetermined pattern (e.g., a stripe pattern in which differently colored straight lines are continuously shown). The pattern of the structured light may be generated, for example, using a pattern mask or a digital micro-mirror device (DMD), but the present disclosure is not limited thereto. The camera 205 of the three-dimensional scanner 200 according to various embodiments may acquire an image of the oral cavity of the subject 20 by receiving light reflected by the oral cavity of the subject 20. The camera 205 may include, for example, a left camera corresponding to the left field of view and a right camera corresponding to the right field of view in order to construct a three-dimensional image by using optical triangulation. The camera 205 may include at least one image sensor, such as a CCD sensor or a CMOS sensor.

The input device 206 of the three-dimensional scanner 200 according to various embodiments may receive a user input for controlling the three-dimensional scanner 200. The input device 206 may include buttons for receiving push manipulation from the user 10, a touch panel for detecting touch from the user 10, and a speech recognition device including a microphone. For example, the user 10 may use the input device 206 to control the start or stop of scanning.

The sensor module 207 of the three-dimensional scanner 200 according to various embodiments may detect an operational state of the three-dimensional scanner 200 or an external environmental state (e.g., a user's motion) and generate an electrical signal corresponding to the detected state. The sensor module 207 may include, for example, at least one of a gyro sensor, an accelerometer, a gesture sensor, a proximity sensor, or an infrared sensor. The user 10 may use the sensor module 207 to control the start or stop of scanning. For example, when the user 10 is moving while holding the three-dimensional scanner 200 in hand, the three-dimensional scanner 200 may be controlled to start a scanning operation of the processor 201 when an angular velocity measured by the sensor module 207 exceeds a predetermined threshold.

According to an embodiment, the three-dimensional scanner 200 may start scanning by receiving a user input for starting scanning through the input device 206 of the three-dimensional scanner 200 or an input device 206 of the electronic device 100, or in response to processing in a processor 201 of the three-dimensional scanner 200 or the processor 201 of the electronic device 100. When the user 10 scans the inside of the oral cavity of the subject 20 by using the three-dimensional scanner 200, the three-dimensional scanner 200 may generate a two-dimensional image of the oral cavity of the subject 20, and may transmit the two-dimensional image of the oral cavity of the subject 20 to the electronic device 100 in real time. The electronic device 100 may display the received two-dimensional image of the oral cavity of the subject 20 on a display. Further, the electronic device 100 may generate (construct) a three-dimensional image of the oral cavity of the subject 20, based on the two-dimensional images of the oral cavity of the subject 20, and may display the three-dimensional image of the oral cavity on the display. The electronic device 100 may display the three-dimensional image, which is being generated, on the display in real time.

The electronic device 100 according to various embodiments may include at least one processor 101, at least one memory 103, a communication circuit 105, a display 107, and/or an input device 109. At least one of the elements included in the electronic device 100 may be omitted, or other elements may be added to the electronic device 100. Additionally or alternatively, some elements may be integrated, or implemented as a single or multiple entities. At least some elements in the electronic device 100 may be connected to each other via a bus, a general purpose input/output (GPIO), a serial peripheral interface (SPI), or a mobile industry processor interface (MIPI) so as to exchange data and/or signals.

According to various embodiments, the at least one processor 101 of the electronic device 100 may be an element capable of performing operations or data processing related to control and/or communication of the elements of the electronic device 100 (e.g., memory 103). The at least one processor 101 may be operatively coupled to the elements of the electronic device 100, for example. The at least one processor 101 may load commands or data received from other elements of the electronic device 100 into the at least one memory 103, may process the commands or data stored in the at least one memory 103, and store the resulting data.

According to various embodiments, the at least one memory 103 of the electronic device 100 may store instructions for operations of the at least one processor 101. The at least one memory 103 may store correlation models constructed based on a machine learning algorithm. The at least one memory 103 may store data (e.g., a two-dimensional image of the oral cavity acquired through oral scanning) received from the three-dimensional scanner 200.

According to various embodiments, the communication circuit 105 of the electronic device 100 may establish a wired or wireless communication channel with an external device (e.g., the three-dimensional scanner 200 or the cloud server), and may transmit or receive various types of data to or from the external device. According to an embodiment, for wired communication with the external device, the communication circuit 105 may include at least one port so as to be connected to the external device through a wired cable. In the above case, the communication circuit 105 may communicate with the external device that is connected to a wire via the at least one port. According to an embodiment, the communication circuit 105 may be configured to include a cellular communication module so as to be connected to a cellular network (e.g., 3G, LTE, 5G, Wibro, or Wimax). According to various embodiments, the communication circuit 105 may include a short-range communication module to transmit and receive data to and from external devices by using short-range communication (e.g., Wi-Fi, Bluetooth, Bluetooth Low Energy (BLE), or UWB), but the present disclosure is not limited thereto. According to an embodiment, the communication circuit 105 may include a contactless communication module for contactless communication. The contactless communication may include at least one contactless proximity communication technology, for example, near-field communication (NFC) communication, radio frequency identification (RFID) communication, or magnetic secure transmission (MST) communication.

The display 107 of the electronic device 100 according to various embodiments may display various screens based on control of the processor 101. The processor 101 may display, on the display 107, a two-dimensional image of the oral cavity of subject 20 received from the three-dimensional scanner 200 and/or a three-dimensional image of the oral cavity in which the inner structure of the oral cavity is modeled. For example, the two-dimensional image and/or the three-dimensional image of the oral cavity may be displayed through a particular application. In the above case, the user 10 may edit, save, and delete the two-dimensional image and/or the three-dimensional image of the oral cavity.

The input device 109 of the electronic device 100 according to various embodiments may receive commands or data, which are to be used by an element (e.g., the at least one processor 101) of the electronic device 100, from an external source (e.g., a user) of the electronic device 100. The input device 109 may include, for example, a microphone, a mouse, or a keyboard. According to an embodiment, the input device 109 may be implemented in the form of a touch sensor panel that may be coupled to the display 107 to recognize contact or proximity of various external objects.

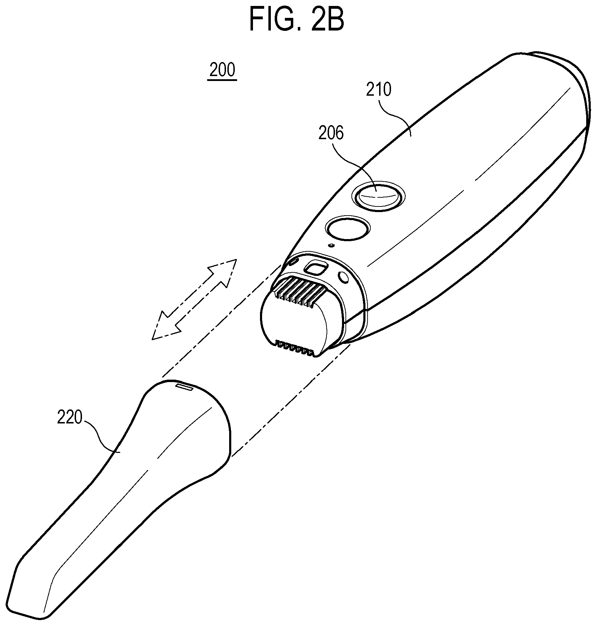

FIG. 2B is a perspective view of a three-dimensional scanner 200 according to various embodiments. The three-dimensional scanner 200 according to various embodiments may include a body 210 and a probe tip 220. The body 210 of the three-dimensional scanner 200 may be formed in a shape that is easy for the user 10 to grip and use by hand. The probe tip 220 may be shaped for easy insertion into and removed from the oral cavity of the subject 20. Further, the body 210 may be coupled to and detachable from the probe tip 220. Inside the body 210, the elements of the three-dimensional scanner 200 illustrated in FIG. 2A may be disposed. An opening may be formed at one end of the body 210 so that light output from the light source 204 can be emitted onto the subject 20 through the opening. Light emitted through the opening may be reflected by the subject 20 and introduced again through the opening. The reflected light introduced through the opening may be captured by the camera to generate an image of the subject 20. The user 10 may start scanning by using the input device 206 (e.g., a button) of the three-dimensional scanner 200. For example, when the user 10 touches or presses the input device 206, light from the light source 204 may be emitted onto the subject 20.

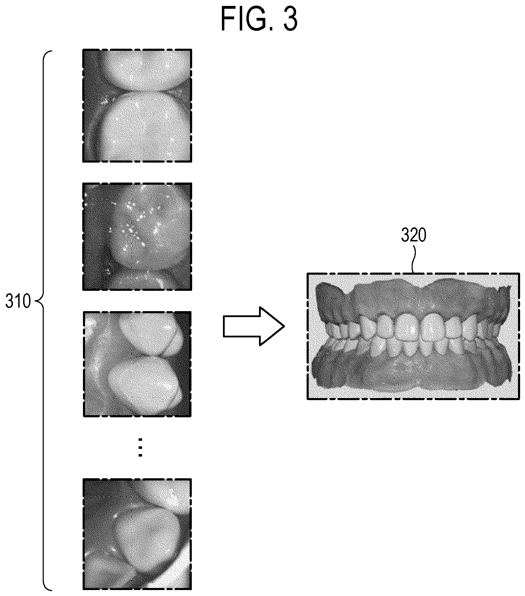

FIG. 3 illustrates a method for generating a three-dimensional image 320 of an oral cavity according to various embodiments. The user 10 may move the three-dimensional scanner 200 to scan the inside of the oral cavity of the subject 20, in which case the three-dimensional scanner 200 may acquire multiple two-dimensional images 310 of the oral cavity of the subject 20. For example, the three-dimensional scanner 200 may acquire a two-dimensional image of a region including a front tooth of the subject 20, a two-dimensional image of a region including a molar of the subject 20, and the like. The three-dimensional scanner 200 may transmit the multiple acquired two-dimensional images 310 to the electronic device 100. According to another embodiment, the user 10 may scan a diagnostic model of the oral cavity while moving the three-dimensional scanner 200, or acquire multiple two-dimensional images of the diagnostic model of the oral cavity. Hereinafter, for convenience of explanation, a case in which an image of the oral cavity of the subject 20 is acquired by scanning the inside of the oral cavity of the subject 20 is assumed, but the embodiments are not limited thereto.

According to various embodiments, the electronic device 100 may convert each of the multiple two-dimensional images 310 of the oral cavity of the subject 20 into a set of multiple points having three-dimensional coordinate values. For example, the electronic device 100 may convert each of the multiple two-dimensional images 310 into a point cloud, which is a set of data points having three-dimensional coordinate values. For example, a set of point clouds, which are three-dimensional coordinate values based on the multiple two-dimensional images 310, may be stored as raw data for the oral cavity of subject 20. By aligning the point clouds, each of which is a set of data points having three-dimensional coordinate values, the electronic device 100 may complete a full dental model.

According to various embodiments, the electronic device 100 may reconfigure (reconstruct) a three-dimensional image of the oral cavity. For example, the electronic device 100 may reconfigure the three-dimensional image 320 of the oral cavity of the subject 20 by merging a set of point clouds stored as raw data by using a Poisson algorithm to reconfigure multiple points and transforming the points into a closed three-dimensional surface.

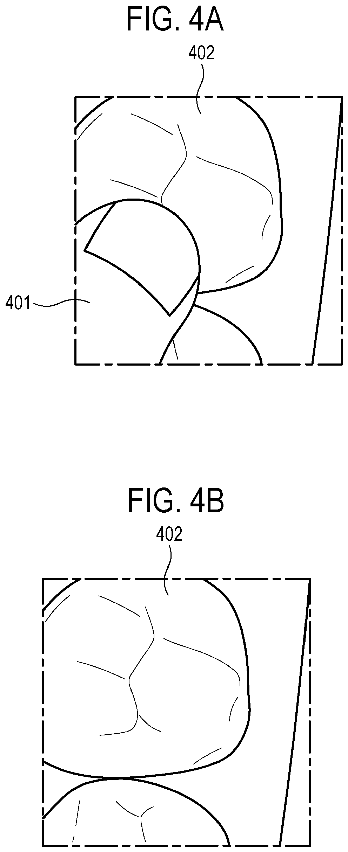

FIGS. 4A to 4E are schematic views illustrating situations in which noise is generated during scanning using a three-dimensional scanner. FIGS. 4A and 4B illustrate two-dimensional images which the three-dimensional scanner 200 acquired by scanning a subject at a first scan time point and a second scan time point, respectively. FIGS. 4A and 4B illustrate a situation in which an obstacle, such as a finger 401 that may cause noise, covers a portion of a tooth 402 at the first scan time point, and a situation in which the obstacle has been removed at the second scan time point subsequent to the first scan time point.

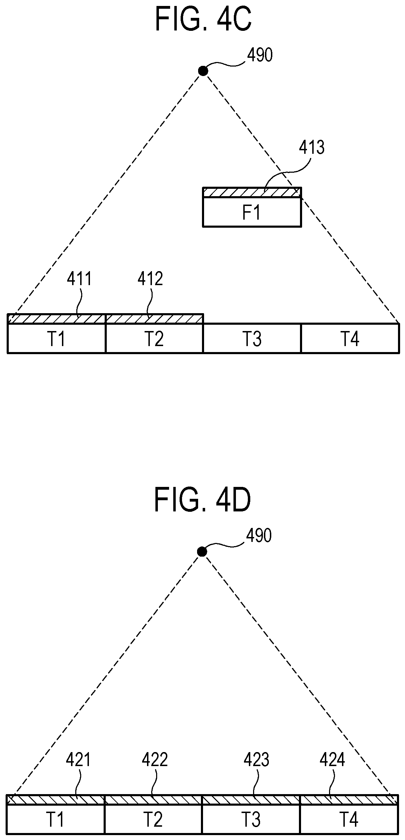

FIGS. 4C and 4D are schematic views illustrating the situations in FIGS. 4A and 4B, respectively. In FIG. 4C, T1 to T4 represent teeth as subjects, and F1 represents an obstacle (e.g., a finger) as a subject. When scanning is performed by the three-dimensional scanner 200, the surfaces of T1 and T2 and the surface of F1, which can be detected by the three-dimensional scanner 200, are scanned. Scanning may be performed through the head portion of the probe tip 220 of the three-dimensional scanner 200. Accordingly, the electronic device 200 generates three-dimensional image models 411, 412, and 413. However, the surfaces of portions T3 and T4 are not scanned due to the obstacle F1. In FIG. 4C, the three-dimensional image models 411, 412, and 413 are illustrated as having a thickness. However, this is for ease of description, and the three-dimensional image models 411, 412, and 413 may correspond to "surfaces." A focal point 490 represents a virtual focal point of the three-dimensional scanner. According to an embodiment, the focal point 490 may be, for example, a position of the light source 204 of the three-dimensional scanner or a position proximate thereto, a position of the camera 205 or a position proximate thereto, or any other positions determined by the geometric relation between these elements.

FIG. 4D illustrates a situation in which there is no obstacle between the teeth T1 to T4 and the three-dimensional scanner 200 at the second scan time point subsequent to the first scan time point. When scanning is performed by the three-dimensional scanner 200, the surfaces of T1, T2, T3, and T4, which are detectable by the three-dimensional scanner 200, are scanned. Accordingly, the electronic device 200 generates three-dimensional image models 421, 422, 423, and 424.

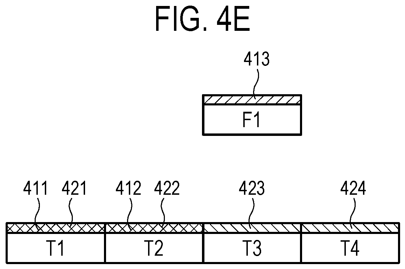

FIG. 4E illustrates a three-dimensional image model generated as a result of the first scanning and the second scanning. As illustrated in FIG. 4C, the generated three-dimensional image model includes a surface 413 corresponding to the obstacle F1 as it is. Thus, even when intact three-dimensional image models 421, 422, 423, and 424 of tooth surfaces are generated by the second scanning, the resulting three-dimensional image models include noise generated due to the surface 413 corresponding to the obstacle, and due to the noise, an accurate three-dimensional image model of a subject corresponding to the tooth may not be generated.

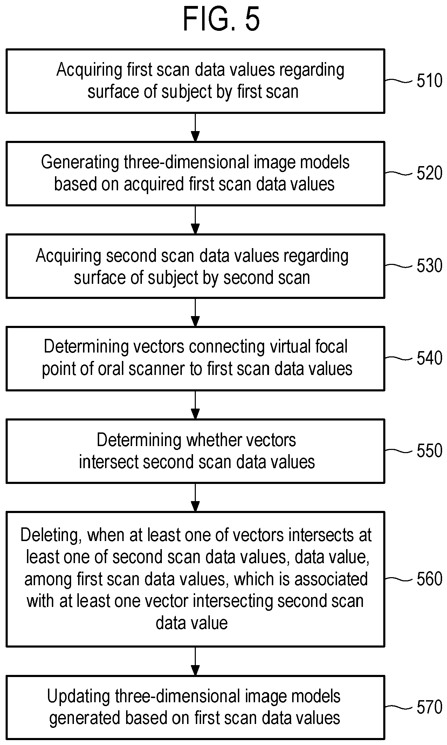

FIG. 5 is a flowchart 500 of a noise filtering method according to various embodiments of the present disclosure. At least a part of the filtering method according to the present disclosure may be a method implemented by a computer, for example, the electronic device 100. However, the filtering method according to the present disclosure is not limited thereto, and may also be implemented by other devices, for example, the three-dimensional scanner 200.

In the illustrated flowcharts, steps of the method according to the present disclosure have been illustrated in a sequential order. However, in addition to being performed sequentially, the steps may be performed in any order in which the steps can be arbitrarily combined by the present disclosure. The description based on the flowchart neither excludes making changes or modifications to the method or algorithm nor implies that any step is essential or desirable. In an embodiment, at least some steps may be performed in parallel, iteratively, or heuristically. In an embodiment, at least some steps may be omitted, or other steps may be added.

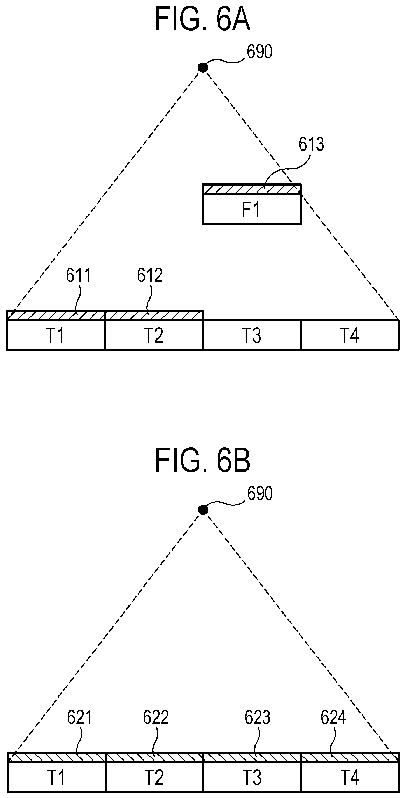

FIGS. 6A to 6D are schematic views illustrating a configuration for filtering noise based on the noise filtering method in FIG. 5. Hereinafter, the noise filtering method in FIG. 5 will be described with reference to FIGS. 6A to 6D.

In step 510, first scan data values regarding the surfaces of subjects are acquired by a first scan. The first scan may correspond to image capturing once performed by a camera of a three-dimensional scanner, or may also correspond to image capturing performed a predetermined number of times necessary to generate three-dimensional image models. In an embodiment, the first scan may correspond to scanning over a predetermined time. In an embodiment, the acquired first scan data values include three-dimensional coordinate values. The three-dimensional coordinate values may be generated based on two-dimensional image data acquired by the scanner. In an embodiment, the first scan data values may include three-dimensional volume data in the form of voxels.

In step 520, three-dimensional image models are generated based on the first scan data values acquired by the first scan. The generated three-dimensional image models may be displayed on the display 107 of the electronic device 100. In an embodiment, an aligning step is further performed to connect and align the generated three-dimensional volume data (e.g., voxels) with each other.

FIG. 6A illustrates a situation in which an obstacle as a subject is interposed between teeth as subjects and the three-dimensional scanner at a first scan time point. In FIG. 6A, T1 to T4 represent, for example, teeth, and F1 represents, for example, a finger that may cause noise. When scanning is performed by the three-dimensional scanner 200, the surfaces of T1 and T2 and the surface of F1, which may be detected by the three-dimensional scanner 200, are scanned, and three-dimensional image models 611, 612, and 613 are generated accordingly. However, the surfaces of T3 and T4 are not scanned due to the obstacle F1. The surfaces 611, 612, and 613 including the first scan data values acquired by the first scan are referred to as "first surfaces" (denoted by 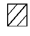 ). A focal point 690 represents the virtual focal point of the three-dimensional scanner.

In step 530, second scan data values regarding the surfaces of the subjects are acquired by a second scan subsequent to the first scan. In an embodiment, the acquired second scan data values include three-dimensional coordinate values. The three-dimensional coordinate values may be generated based on the two-dimensional image data acquired by the scanner. In an embodiment, the second scan data values may be three-dimensional volume data in the form of voxels.

FIG. 6B illustrates a situation in which the obstacle between the subjects (the teeth) and the three-dimensional scanner has been removed at a second scan time point. For ease of description, it is assumed that areas being scanned during the first scan and the second scan are the same. That is, it is assumed that the probe tip 220 of the three-dimensional scanner 200 is not moved during the first scan and the second scan. When scanning is performed by the three-dimensional scanner 200, the surfaces of T1, T2, T3, and T4, which may be detected by the three-dimensional scanner 200, are scanned, and three-dimensional image models 621, 622, 623, and 624 are generated accordingly. The surfaces 621, 622, 623, 624 including the second scan data values acquired by the second scan are referred to as "second surfaces" (denoted by 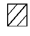 ).

In step 540, to filter out noise that may have been generated by the first scan, virtual vectors connecting the virtual focal point 690 of the three-dimensional scanner 200 to the first scan data values, respectively, are determined. The vectors may extend from the virtual focal point 690 to pass through the first scan data values, respectively. In an embodiment, the vectors extend from the virtual focal point to pass through voxel values, corresponding to the first scan data values, respectively.

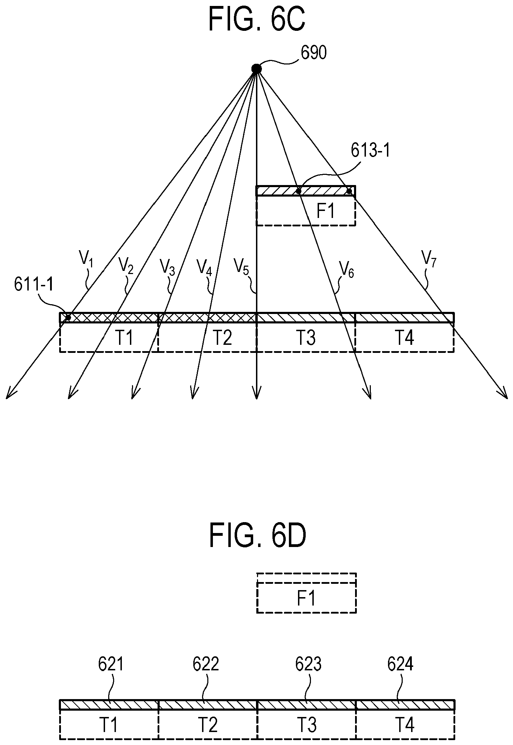

FIG. 6C illustrates vectors V1 to V7 extending from the virtual focal point 690 to pass through the first scan data values, respectively. For example, the vector V1 is a vector extending from the virtual focal point 690 to pass through a first scan data value 611-1, and the vector V6 is a vector extending from the virtual focal point 690 to pass through a first scan data value 613-1. Although only seven vectors V1 to V7 are illustrated in FIG. 6C for convenience, vectors may be generated for all of the first scan data values.

In step 550, it is determined whether the determined vectors intersect the acquired second scan data values. In an embodiment, determining whether the vectors intersect the second scan data values includes determining whether the vectors intersect the second surfaces. In another embodiment, determining whether the vectors intersect the second scan data values includes determining whether the distance between each of the vectors and a second scan data value at the closest vertical distance is equal to or less than a predetermined threshold value. In step 560, when at least one of the vectors intersects at least one of the second scan data values, a first scan data value associated with the vector that intersects the second scan data value is considered to be noise and is deleted from first scan data.

In the embodiment illustrated in FIG. 6C, the vectors V1 to V5 intersect the second surface 621 and 622 comprising the second scan data values. The vectors V6 and V7 intersect the second surface 623 and 624 comprising the second scan data values. As such, the first scan data values associated with the vectors V1 to V7 intersecting the second scan data values or the second surfaces are considered to be noise and are deleted from the first scan data values. As a result, the scan data values of the first surfaces 611, 612, and 613 associated with the vectors V1 to V7 are deleted.

In step 570, the three-dimensional image models of the first scan data values are updated based on the first scan data values excluding the data values that have been considered to be noise and have been deleted. In an embodiment, the first scan data values are three-dimensional volume data in the form of voxels, and updating the three-dimensional image models of the first scan data values includes removing, from the three-dimensional image models, volumes corresponding to the voxels that have been considered to be noise and have been deleted.

FIG. 6D illustrates three-dimensional image models resulting from filtering based on the noise filtering method (steps 610 to 670) according to various embodiments of the present disclosure. As a result of the deletion of the first scan data values associated with the vectors V1 to V7 intersecting the second scan data values or the second surfaces, that is, the values corresponding to the first surfaces 611, 612, and 613, only the second surfaces 621 to 624 remain in the updated three-dimensional image models. In this way, it is possible to filter out surface data of the obstacle F1 corresponding to noise while maintaining surface data of the subjects T1 to T4.

In the above embodiment of FIGS. 5 and 6A to 6D, data values, among the first scan data values, which correspond to noise have been deleted by determining vectors connecting the virtual focal point to the first scan data values and by determining whether the vectors intersect the second scan data values. However, the present disclosure is not limited thereto. In another embodiment of the present disclosure, data values, among the first scan data values, which correspond to noise may be deleted by determining vectors connecting the virtual focal point to the second scan data values and by determining whether the vectors intersect the first scan data values.

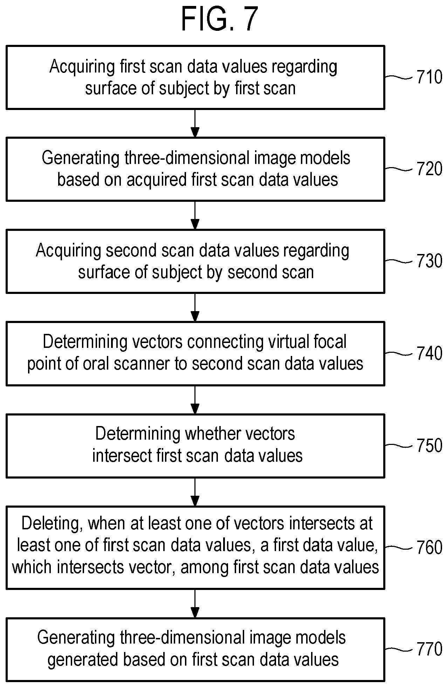

Specifically, FIG. 7 is a flowchart 700 of a noise filtering method according to various embodiments of the present disclosure.

In step 710, first scan data values for the surfaces of subjects are acquired by a first scan. The first scan may correspond to image capturing once performed by a camera of a three-dimensional scanner, and may also correspond to image capturing performed a predetermined number of times necessary to generate three-dimensional image models. In an embodiment, the first scan may correspond to scanning over a predetermined time. In an embodiment, the acquired first scan data values include three-dimensional coordinate values. The three-dimensional coordinate values may be generated based on two-dimensional image data acquired by the scanner. In an embodiment, the first scan data values may include three-dimensional volume data in the form of voxels.

In step 720, three-dimensional image models are generated based on the first scan data values acquired by the first scan. The generated three-dimensional image models may be displayed on the display 107 of the electronic device 100. In an embodiment, an align step is further performed to connect and align the generated three-dimensional volume data (e.g., voxels) with each other.

In step 730, second scan data values regarding the surfaces of the subjects are acquired by a second scan subsequent to the first scan. In an embodiment, the acquired second scan data values include three-dimensional coordinate values. The three-dimensional coordinate values may be generated based on the two-dimensional image data acquired by the scanner. In an embodiment, the second scan data values may be three-dimensional volume data in the form of voxels.

In step 740, to filter out noise that may have been generated by the second scan, virtual vectors connecting a virtual focal point of the three-dimensional scanner 200 to the second scan data values, respectively, are determined. The vectors may extend from the virtual focal point to pass through the second scan data values, respectively. In an embodiment, the vectors extend from the virtual focal point to pass through voxel values, corresponding to the second scan data values, respectively.

In step 750, it is determined whether the determined vectors intersect the acquired first scan data values. In an embodiment, determining whether the vectors intersect the first scan data values includes determining whether the vectors intersect the first surfaces. In another embodiment, determining whether the vectors intersect the first scan data values includes determining whether the distance between each of the vectors and a first scan data value at the closest vertical distance is equal to or less than a predetermined threshold value. In step 760, when at least one of the vectors intersects at least one of the first scan data values, a first scan data value that intersects the at least one of the vectors is considered to be noise and is deleted from the first scan data.

In step 770, three-dimensional image models of the first scan data values are generated based on the first scan data values excluding the data values that have been considered to be noise and have been deleted.

In the embodiments of the present disclosure, it has been described that vectors connecting a focal point to each piece of scan data are determined and the first scan data values corresponding to noise are deleted based on whether the vectors intersect pieces of the scan data. However, the embodiments of the present disclosure may be applied to remove second scan data values corresponding to noise based on whether the vectors intersect the scan data.

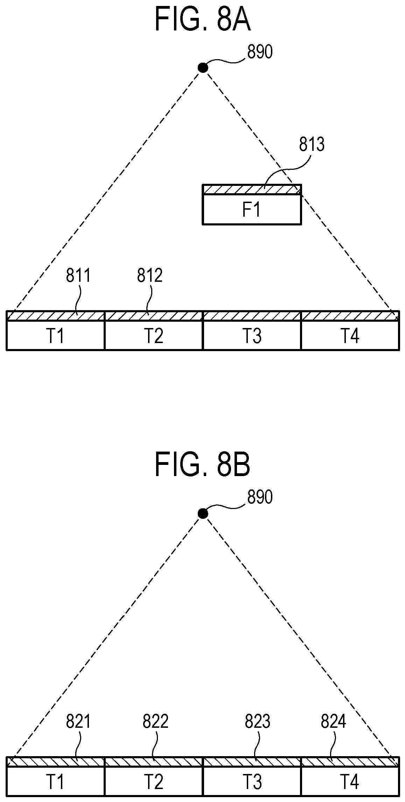

FIGS. 8A to 8D illustrate a noise filtering method according to various embodiments of the present disclosure. For ease of description, detailed descriptions of configurations that overlap with FIGS. 6A to 6D will be omitted.

In FIG. 8A, T1 to T4 represent subjects (e.g., teeth), and F1 represents an obstacle (e.g., a finger) that may cause noise. When scanning is performed by the three-dimensional scanner 200, the surfaces of T1 and T2 and the surface of F1, which may be detected by the three-dimensional scanner 200, are scanned, and three-dimensional image models 811, 812, and 813 are generated accordingly. The surfaces 811, 812, and 813 including first scan data values acquired by a first scan are referred to as "first surfaces" (denoted by 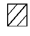 ). A focal point 890 represents the virtual focal point of the three-dimensional scanner.

In FIG. 8B, when scanning is performed by the three-dimensional scanner 200, the surfaces of T1, T2, T3, and T4, which may be detected by the three-dimensional scanner 200, are scanned at a second scan time point, and three-dimensional image models 821, 822, 823, and 824 are generated accordingly. The surfaces 821, 822, 823, 824 including second scan data values acquired by a second scan are referred to as "second surfaces" (denoted by 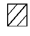 ).

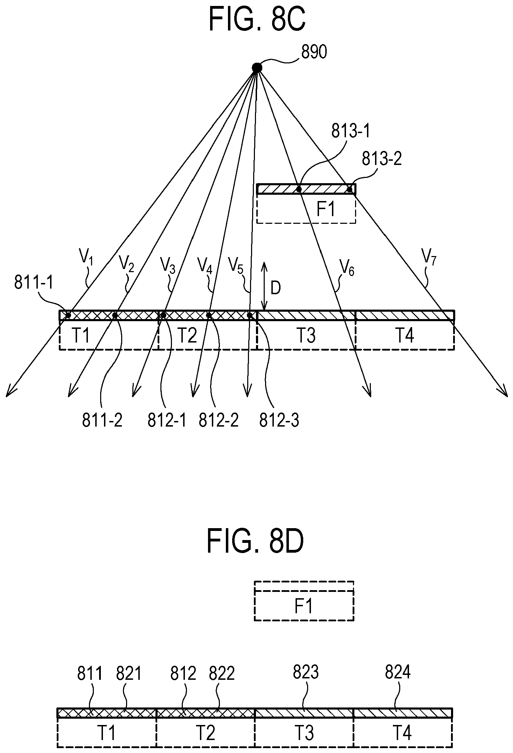

FIG. 8C illustrates vectors V1 to V7 extending from the virtual focal point 890 to pass through the first scan data values, respectively, according to various embodiments of the present disclosure. For example, the vector V1 is a vector extending from the virtual focal point 890 to pass through a first scan data value 811-1, and the vector V6 is a vector extending from the virtual focal point 890 to pass through a first scan data value 813-1. Although only seven vectors V1 to V7 are illustrated in FIG. 8C for convenience, vectors may be generated for all of the first scan data values.

Thereafter, it is determined whether the generated vectors intersect the acquired second scan data values or the second surfaces. As in the embodiment illustrated in FIG. 6C, the vectors V1 to V5 intersect the second surfaces 821 and 822 comprising the first scan data values, and the vectors V6 andV7 intersect the second surfaces 823 and 824 comprising the second scan data values. However, in the embodiment illustrated in FIG. 8C, when a second scan data value intersected by a vector is located within a predetermined distance D from a first scan data value associated with that vector, the vector is determined not to have intersected the second scan data value. In other words, only when a second scan data value intersected by a vector and a first scan data value associated with that vector are farther apart than the predetermined distance D, the vector is determined to have intersected the second scan data value. The predetermined distance D may be a distance in a preset three-dimensional coordinate space.

In the embodiment illustrated in FIG. 8C, it may be found that the first scan data value 811-1 associated with the vector V1 intersecting the second surface 821 is on or very close to the second surface 821. That is, the distance between a second scan data value on the second surface 821 intersected by the vector V1 and the first scan data value 811-1 associated with the vector V1 is within the predetermined distance D. In this case, the vector V1 is not determined to have intersected the second scan data value or the second surface 821. As in the case of the vector V1, the distances between second scan data values on the second surfaces 821 and 822 intersected by the vectors V2 to V5 and first scan data values 811-2, 812-1, 812-2, and 812-3 associated with the vectors V2 through V5 are within the predetermined distance D, and thus the vectors V2 to V5 are not determined to have intersected the second scan data values or the second surfaces.

On the other hand, the distance between a second scan data value on the second surface 823 intersected by the vector V6 and the first scan data value 813-1 associated with the vector V6 is greater than the predetermined distance D. In this case, the vector V6 is determined to have intersected the second scan data value or the second surface 823. As in the case of the vector V6, the distance between a second scan data value on the second surface 824 intersected by the vector V7 and the first scan data value 813-2 associated with the vector V7 is greater than the predetermined distance D, and thus the vector V7 is determined to have intersected the second scan data value or the second surface.

As described in connection with steps 560 and 570, first scan data values associated with vectors intersecting at least one of the second scan data values are considered to be noise, and are deleted from first scan data, and the three-dimensional image models are updated. In an embodiment, the first scan data values are three-dimensional volume data in the form of voxels, and updating the three-dimensional image models of the first scan data values includes removing, from the three-dimensional image models, volumes corresponding to voxels that have been considered be noise and have been deleted. FIG. 8D illustrates three-dimensional image models resulting from the filtering. The first scan data values associated with the vectors V6 and V7 intersecting the second scan data values or the second surfaces, i.e., the first scan data values corresponding to the first surface 813, are deleted, while the first surfaces 811 and 812 and the second surfaces 821 to 824 are maintained. In this way, by determining whether to perform filtering, based on the distance between a second scan data value intersected by a vector and a first scan data value associated with the vector, it is possible to more precisely filter out a scan data value that corresponds to actual noise. In addition, since the first scan data values in similar positions to the second scan data values are maintained, it is possible to utilize the first scan data values for alignment with the second scan data values to construct more accurate three-dimensional image models.

In the above-described embodiments of the present disclosure, vectors are generated for all of the first scan data values to determine whether the vectors intersect the second scan data values (or the second surfaces). This configuration has an advantage of detecting all scan data values that are likely to be noise generated during the first scan, but generating vectors for all of the first scan data values and determining intersection for each of the vectors has a disadvantage of causing a long processing time or using a lot of data unnecessarily. This may impose an excessive load on the processor of the electronic device 100, thereby adversely affecting a scanning operation and the generation of a three-dimensional image model.

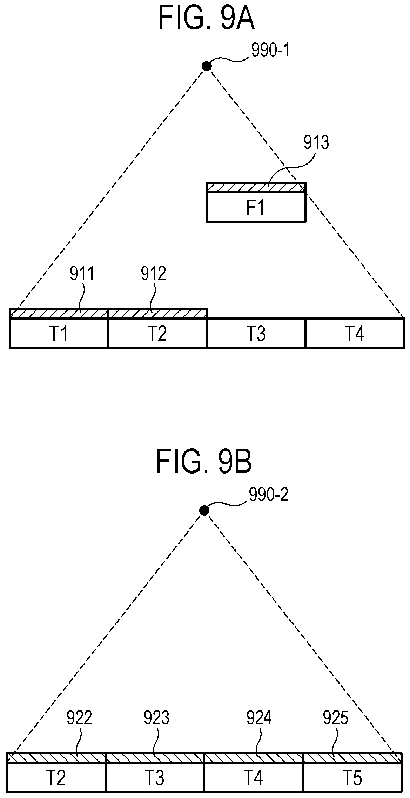

To resolve this, FIGS. 9A to 9D illustrate a noise filtering method according to various embodiments of the present disclosure. For ease of description, detailed descriptions of configurations that overlap with FIGS. 8A to 8D will be omitted. The embodiments illustrated in FIGS. 6A to 6D and FIGS. 8A to 8D assumed that the position of the scanner head during the first and second scans is the same. However, the present embodiment assumes that the probe tip 220 of the three-dimensional scanner 200 is moved to the right by the width of the subject T3.

In FIG. 9A, T1 to T4 represent subjects (e.g., teeth), and F1 represents an obstacle (e.g., a finger) that may cause noise. When scanning is performed by the three-dimensional scanner 200, the surfaces of T1 and T2 and the surface of F1, which may be detected by the three-dimensional scanner 200, are scanned, and three-dimensional image models 911, 912, and 913 are generated accordingly. The surfaces 911, 912, and 913 including first scan data values acquired by a first scan are referred to as "first surfaces" (denoted by 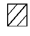 ). A focal point 990-1 represents the virtual focal point of the three-dimensional scanner at a first scan time point.

In FIG. 9B, the probe tip 220 of the three-dimensional scanner 200 moves to the right at a second scan time point, so that the surfaces of T2, T3, T4, and T5 are scanned, and three-dimensional image models 922, 923, 924, and 925 are generated accordingly. The surfaces 922, 923, 924, and 925 including second scan data values acquired by a second scan are referred to as "second surfaces" (denoted by 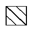 ). A focal point 990-2 represents the virtual focal point of the three-dimensional scanner at the first scan time point. As described above, the focal point 990-2 is located to the right of the focal point 990-1 by the width of the subject T3

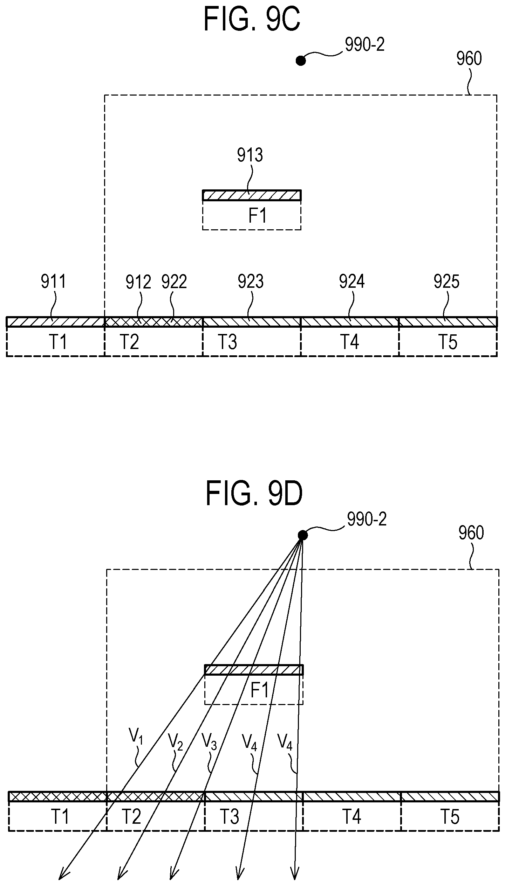

In FIG. 9C, a virtual volume 960 is set to include all of the second scan data values. The virtual volume 960 may also be referred to as a bounding box. The bounding box 960 may correspond to a volume space formed in a hexahedral, cylindrical, conic, or any other three-dimensional shape that includes all of the second scan data values.

In FIG. 9D, vectors are generated only for first scan data values included in the bounding box 960. That is, vectors V1 to V5 extending through the first scan data values from the virtual focal point 990-2 are determined only for first scan data values in the first surfaces 912 and 913 included in the bounding box 960, and no vectors are determined for first scan data values in the first surface 911 which is not included in the bounding box 960. When the vectors are determined, it is determined whether the determined vectors intersect the second scan data values (or the second surfaces), as described in connection with steps 550 to 570. When at least one of the vectors intersects at least one of the second scan data values, a first scan data value associated with the intersected vector is considered to be noise and is deleted from first scan data, and the three-dimensional image models are updated.

In the embodiments illustrated in FIGS. 9A through 9D, in determining whether a determined vector intersects a second scan data value (or a second surface), the vector may be determined not to have intersected the second scan data value when, as described in connection with FIGS. 8A to 8D, the second scan data value intersected by the vector is located within a predetermined distance from a first scan data value associated with the vector.

Also, FIG. 9D illustrates an embodiment in which vectors are generated based on the virtual focal point 990-2 at the second scan time point, but vectors may also be generated based on the virtual focal point 990-1 at the first scan time point.

In the above embodiments in FIGS. 8A to 8D and 9A to 9D, data values corresponding to noise among the first scan data values are deleted by determining vectors connecting the virtual focal point to the first scan data values and determining whether the vectors intersect the second scan data values. However, as described in connection with FIG. 7, it is also possible to delete data values corresponding to noise, among the first scan data values, by determining vectors connecting the virtual focal point to the second scan data values and determining whether the vectors intersect the first scan data values.

In addition to using the bounding box, other methods may be used to reduce the number of vectors generated. For example, scan data values that are relatively likely to be noise may be selected, and vectors may be generated for only those scan data values and whether they intersect is determined.

In an embodiment, based on the distance between the virtual focal point and each of the first scan data values acquired by the first scan, first scan data values for which vectors are to be generated may be determined. Specifically, vectors may be generated for only first scan data values, which are within a predetermined distance from the virtual focal point, among the first scan data values acquired by the first scan. Referring again to FIG. 6A, vectors may be generated for only the surface 613, which is located within a predetermined distance from the virtual focal point 690, among the first surfaces 611, 612, and 613 including the first scan data values acquired by the first scan, and vectors may not be generated for the other surfaces 611 and 612. This is because in most cases, an obstacle is interposed between a desired subject and a scanner head, so when the distance between a scanned data value and a virtual focal point is short, the data value is likely to be noise. In this way, the number of vectors that need to be generated may be reduced.

Additionally or alternatively, vectors may be generated only for scan data values that have relatively short distances from the virtual focal point.

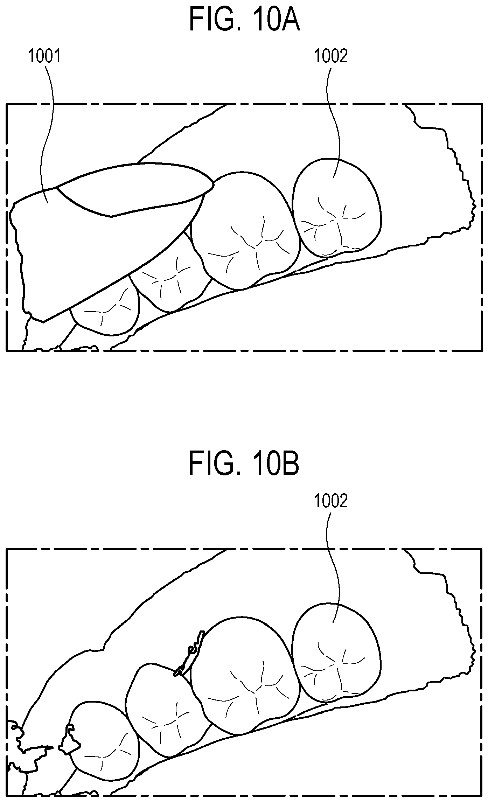

FIGS. 10A and 10B illustrate results of noise filtering performed according to various embodiments of the present disclosure. FIG. 10A is the result of three-dimensional image modeling based on scan data values that include a finger at a first scan time point. In FIG. 10A, a finger portion 1001 corresponds to noise. FIG. 10B illustrates that in subsequent scans, the noise filtering according to various embodiments of the present disclosure has been performed, and the finger that was modeled as a result of a first scan has been mostly filtered out. As illustrated in FIG. 10B, the noise filtering of the present disclosure may effectively remove noise, and noise may be further removed by performing the subsequent scans.

Various embodiments of the present disclosure may be implemented as software recorded in a machine-readable recording medium. The software may be software for implementing the above-mentioned various embodiments of the present disclosure. The software may be inferred from various embodiments of the present disclosure by programmers in a technical field to which the present disclosure belongs. For example, the software may be a machine-readable command (e.g., code or a code segment) or program. A machine may be a device capable of operating according to an instruction called from the recording medium, and may be, for example, a computer. In an embodiment, the machine may be the device 100 according to embodiments of the present disclosure. In an embodiment, a processor of the machine may execute a called command to cause elements of the machine to perform a function corresponding to the command. In an embodiment, the processor may be the at least one processor 101 according to embodiments of the present disclosure. The recording medium may refer to any type of recording medium which stores data capable of being read by the machine. The recording medium may include, for example, ROM, RAM, a CD-ROM, a magnetic tape, a floppy disk, an optical data storage device, and the like. In an embodiment, the recording medium may be the at least one memory 103. In an embodiment, the recording medium may be distributed to computer systems which are connected to each other through a network. The software may be distributed, stored, and executed in the computer systems. The recording medium may be a non-transitory recording medium. The non-transitory recording medium refers to a tangible medium that exists irrespective of whether data is stored semi-permanently or temporarily, and does not include a transitorily transmitted signal.

Although the technical idea of the present disclosure has been described by the examples described in some embodiments and illustrated in the accompanying drawings, it should be noted that various substitutions, modifications, and changes can be made without departing from the technical scope of the present disclosure which can be understood by those skilled in the art to which the present disclosure pertains. In addition, it should be noted that such substitutions, modifications, and changes are intended to fall within the scope of the appended claims.

## Claims

1. A method for processing a scan image of a three-dimensional scanner, which is performed by at least one processor of an electronic device comprising the at least one processor and at least one memory configured to store instructions to be executed by the at least one processor, the method comprising:
acquiring first scan data values regarding a surface of a subject by a first scan of the three-dimensional scanner, the first scan data values comprising three-dimensional coordinate values;
acquiring second scan data values regarding the surface of the subject by a second scan of the three-dimensional scanner, the second scan data values comprising three-dimensional coordinate values;
determining first vectors connecting a virtual focal point of the three-dimensional scanner to the first scan data values or second vectors connecting the virtual focal point to the second scan data values;
determining whether the first vectors intersect the second scan data values or whether the second vectors intersect the first scan data values; and
in case that at least one of the first vectors intersects at least one of the second scan data values, deleting a data value, among the first scan data values, which is associated with the at least one first vector intersecting the at least one second scan data value, and in case that at least one of the second vectors intersects at least one of the first scan data values, deleting a data value, among the first scan data values, which intersects the at least one second vector.

2. The method of claim 1, further comprising:
generating a three-dimensional image model based on the acquired first scan data values after the acquiring the first scan data values; and
updating the generated three-dimensional image model based on the deleted data values after the deleting the data values.

3. The method of claim 2, wherein the first scan data values comprise at least one voxel, and
wherein the updating the generated three-dimensional image model comprises removing, from three-dimensional images associated with the at least one voxel, a three-dimensional image associated with a voxel corresponding to a data value associated with the at least one first vector or a data value intersecting the at least one second vector.

4. The method of claim 1, wherein the determining whether the first vectors intersect the second scan data values or whether the second vectors intersect the first scan data values comprises:
determining that, in case that at least one of the first vectors intersects a surface comprising the second scan data values, the at least one first vector intersects at least one of the second scan data values; and
determining that, in case that at least one of the second vectors intersects a surface comprising the first scan data values, the at least one second vector intersects at least one of the first scan data values.

5. The method of claim 1, wherein the determining whether the first vectors intersect the second scan data values or whether the second vectors intersect the first scan data values comprises:
determining that, in case that a distance between at least one of the first vectors and at least one of the second scan data values is within a threshold value, the at least one first vector intersects the at least one second scan data value; and
determining that, in case that a distance between at least one of the second vectors and at least one of the first scan data values is within a threshold value, the at least one second vector intersects the at least one first scan data value.

6. The method of claim 4 or 5, wherein the determining whether the first vectors intersect the second scan data values or whether the second vectors intersect the first scan data values comprises:
determining that the at least one second scan data value has not intersected the at least one first vector in case that the at least one second scan data value is located within a predetermined distance from a first scan data value, which is associated with the at least one first vector, among the first scan data values; and
determining that the at least one first scan data value has not intersected the at least one second vector in case that the at least one first scan data value is located within a predetermined distance from a second scan data value, which is associated with the at least one second vector, among the second scan data values.

7. The method of claim 1, wherein the determining first vectors connecting a virtual focal point of the three-dimensional scanner to the first scan data values or second vectors connecting the virtual focal point to the second scan data values comprises:
setting a virtual volume comprising all of the second scan data values; and
determining first vectors connecting the virtual focal point of the three-dimensional scanner to data values, which are included in the virtual volume, among the first scan data values, or second vectors connecting the virtual focal point of the three-dimensional scanner to data values, which are included in the virtual volume, among the second scan data values.

8. The method of claim 7, wherein the virtual volume is formed in a hexahedral, cylindrical, conic, or arbitrary three-dimensional shape.

9. The method of claim 1, wherein the determining first vectors connecting a virtual focal point of the three-dimensional scanner to the first scan data values or second vectors connecting the virtual focal point to the second scan data values comprises determining first vectors connecting the virtual focal point of the three-dimensional scanner to data values, each of which has a distance from the virtual focal point within a predetermined value, among the first scan data values, or second vectors connecting the virtual focal point of the three-dimensional scanner to data values, each of which has a distance from the virtual focal point within a predetermined value, among the second scan data values.

10. An electronic device comprising:
a communication circuit communicatively connected to a three-dimensional scanner;
a display; and
at least one processor,
wherein the at least one processor is configured to:
acquire first scan data values regarding a surface of a subject by a first scan of the three-dimensional scanner, the first scan data values comprising three-dimensional coordinate values;
acquire second scan data values regarding the surface of the subject by a second scan of the three-dimensional scanner, the second scan data values comprising three-dimensional coordinate values;
determine first vectors connecting a virtual focal point of the three-dimensional scanner to the first scan data values or second vectors connecting the virtual focal point to the second scan data values;
determine whether the first vectors intersect the second scan data values or whether the second vectors intersect the first scan data values; and
in case that at least one of the first vectors intersects at least one of the second scan data values, delete a data value, among the first scan data values, which is associated with the at least one first vector intersecting the at least one second scan data value, and in case that at least one of the second vectors intersects at least one of the first scan data values, delete a data value, among the first scan data values, which intersects the at least one second vector.

11. The electronic device of claim 10, wherein the at least one processor is configured to:
generate, after acquiring the first scan data values, a three-dimensional image model based on the acquired first scan data values; and
update, after deleting the data values, the generated three-dimensional image model based on the deleted data values.

12. The electronic device of claim 11, wherein the first scan data values comprise at least one voxel, and
wherein the updating of the generated three-dimensional image model comprises removing, from three-dimensional images associated with the at least one voxel, a three-dimensional image associated with a voxel corresponding to a data value associated with the at least one first vector or a data value intersecting the at least one second vector.

13. The electronic device of claim 10, wherein the determining of whether the first vectors intersect the second scan data values or whether the second vectors intersect the first scan data values comprises:
determining that, in case that at least one of the first vectors intersects a surface comprising the second scan data values, the at least one vector intersects at least one of the second scan data values; and
determining that, in case that at least one of the second vectors intersects a surface comprising the first scan data values, the at least one second vector intersects at least one of the first scan data values.

14. The electronic device of claim 10, wherein the determining of whether the first vectors intersect the second scan data values or whether the second vectors intersect the first scan data values comprises:
determining that, in case that a distance between at least one of the first vectors and at least one of the second scan data values is within a threshold value, the at least one first vector intersects the at least one second scan data value; and
determining that, in case that a distance between at least one of the second vectors and at least one of the first scan data values is within a threshold value, the at least one second vector intersects the at least one first scan data value.

15. The electronic device of claim 13 or 14, wherein the determining of whether the first vectors intersect the second scan data values or whether the second vectors intersect the first scan data values comprises:
determining that the at least one second scan data value has not intersected the at least one first vector in case that the at least one second scan data value is located within a predetermined distance from a first scan data value, which is associated with the at least one first vector, among the first scan data values; and
determining that the at least one first scan data value has not intersected the at least one second vector in case that the at least one first scan data value is located within a predetermined distance from a second scan data value, which is associated with the at least one second vector, among the second scan data values.

16. The electronic device of claim 10, wherein the determining of first vectors connecting a virtual focal point of the three-dimensional scanner to the first scan data values or second vectors connecting the virtual focal point to the second scan data values comprises:
setting a virtual volume comprising all of the second scan data values; and
determining first vectors connecting the virtual focal point of the three-dimensional scanner to data values, which are included in the virtual volume, among the first scan data values, or second vectors connecting the virtual focal point of the three-dimensional scanner to data values, which are included in the virtual volume, among the second scan data values.

17. The electronic device of claim 16, wherein the virtual volume is formed in a hexahedral, cylindrical, conic, or arbitrary three-dimensional shape.

18. The electronic device of claim 10, wherein the determining of first vectors connecting a virtual focal point of the three-dimensional scanner to the first scan data values or second vectors connecting the virtual focal point to the second scan data values comprises determining first vectors connecting the virtual focal point of the three-dimensional scanner to data values, each of which has a distance from the virtual focal point within a predetermined value, among the first scan data values, or second vectors connecting the virtual focal point of the three-dimensional scanner to data values, each of which has a distance from the virtual focal point within a predetermined value, among the second scan data values.

19. A non-transitory computer-readable recording medium storing instructions which, when executed by at least one processor, cause the at least one processor to perform operations, wherein the instructions cause the at least one processor to:
acquire first scan data values regarding a surface of a subject by a first scan of a three-dimensional scanner, the first scan data values comprising three-dimensional coordinate values;
acquire second scan data values regarding the surface of the subject by a second scan of the three-dimensional scanner, the second scan data values comprising three-dimensional coordinate values;
determine first vectors connecting a virtual focal point of the three-dimensional scanner to the first scan data values or second vectors connecting the virtual focal point to the second scan data values;
determine whether the first vectors intersect the second scan data values or whether the second vectors intersect the first scan data values; and
in case that at least one of the first vectors intersects at least one of the second scan data values, delete a data value, among the first scan data values, which is associated with the at least one first vector intersecting the at least one second scan data value, and in case that at least one of the second vectors intersects at least one of the first scan data values, delete a data value, among the first scan data values, which intersects the at least one second vector.

20. A system for three-dimensional scanning, the system comprising:
a three-dimensional scanner configured to scan a shape of an oral cavity; and
an electronic device communicably coupled to the three-dimensional scanner,
wherein the electronic device comprises:
a communication circuit communicatively connected to the three-dimensional scanner; and
at least one processor,
wherein the at least one processor is configured to:
acquire first scan data values regarding a surface of a subject by a first scan of the three-dimensional scanner, the first scan data values comprising three-dimensional coordinate values;
acquire second scan data values regarding the surface of the subject by a second scan of the three-dimensional scanner, the second scan data values comprising three-dimensional coordinate values;
determine first vectors connecting a virtual focal point of the three-dimensional scanner to the first scan data values or second vectors connecting the virtual focal point to the second scan data values;
determine whether the first vectors intersect the second scan data values or whether the second vectors intersect the first scan data values; and
in case that at least one of the first vectors intersects at least one of the second scan data values, delete a data value, among the first scan data values, which is associated with the at least one first vector intersecting the at least one second scan data value, and in case that at least one of the second vectors intersects at least one of the first scan data values, delete a data value, among the first scan data values, which intersects the at least one second vector.
